# EUROPÄISCHE PATENTSCHRIFT

(11) **EP 2 079 329 B1**
(45) Veröffentlichungstag und Bekanntmachung des Hinweises auf die Patenterteilung: **17.08.2011**
(21) Anmeldenummer: 07818682.2
(22) Anmeldetag: 04.10.2007
(51) Int. Cl.: A24F 47/00, A61M 11/04, A61M 15/06

(54) **INHALATIONSVORRICHTUNG UND HEIZEINRICHTUNG HIERFÜR**
INHALATION DEVICE AND HEATING UNIT THEREFOR
DISPOSITIF D'INHALATION ET UNITÉ DE CHAUFFAGE À CET EFFET

(30) Priorität: 06.10.2006 DE 102006048325; 06.06.2007 DE 102007026979
(43) Veröffentlichungstag der Anmeldung: 22.07.2009
(73) Patentinhaber: Siller, Friedrich, 8103 Unterengstringen (CH)
(72) Erfinder: Siller, Friedrich, 8103 Unterengstringen (CH)
(74) Vertreter: Patentanwälte Ruff, Wilhelm, Beier, Dauster & Partner
(86) Internationale Anmeldenummer: PCT/EP2007/008603
(87) Internationale Veröffentlichungsnummer: WO 2008/043474

(56) Entgegenhaltungen:
- WO-A-2006/082571
- DE-A1- 19 854 008
- GB-A- 1 047 113
- US-A- 4 036 224
- US-B2- 6 598 607

## Beschreibung

### Anwendungsgebiet und Stand der Technik

Die Erfindung betrifft eine Heizeinrichtung für eine Inhalationsvorrichtung zur inhalativen Einnahme eines Inhalationsgemisches aus Luft und mindestens einem Zusatzstoff mit einem Brennstoffspeicher, der mit einem thermisch brennbaren festen oder flüssigen Brennstoff befüllbar oder befüllt ist, und einer Brennkammer zur Verbrennung des Brennstoffs, die von der Umgebung durch eine Brennkammerwandung weitgehend abgeschlossen ist. Die Erfindung betrifft weiterhin eine Inhalationsvorrichtung zur inhalativen Einnahme eines Inhalationsgemisches aus Luft und mindestens einem Zusatzstoff mit einem Mundstück, einem Inhalationsgemischerzeuger mit einer Lufteinlassöffnung und einer Heizeinrichtung zur Verbrennung eines Brennstoffs und zur Erhitzung der Luft und/oder des Zusatzstoffs und/oder des Inhalationsgemisches, wobei der Inhalationsgemischerzeuger mit dem Mundstück unmittelbar oder über einen Inhalationskanal verbunden ist.

Gattungsgemäße Inhalationsvorrichtungen sind aus dem Stand der Technik bekannt. Die Inhalationsvorrichtungen dienen insbesondere der Substitution normalen Tabakkonsums in Form von Zigaretten oder anderen Tabakwaren. Sie können darüber hinaus insbesondere bei der Entwöhnung von Rauchern verwendet werden. Dabei dienen gattungsgemäße Heizeinrichtungen zur Erwärmung der Luft oder der Zusatzstoffe.

Aus der US 5,099,861 A, der US 4,969,476 A sowie der EP 0472367 A1 sind Inhalationsvorrichtungen bekannt, bei denen die Wärmeenergie durch ein glimmendes Kohleelement ohne Flamme zur Verfügung gestellt wird, wobei die entstehenden Verbrennungsgase durch den Benutzer mit inhaliert werden. Aus der DE 2704218 A1 ist ein Gerät bekannt, bei welchem ein Heizelement durch die Flamme einer Heizquelle, beispielsweise eines Feuerzeugs erhitzt wird. Aus der US 6,164,287 A und der JP 2000 236 865 A sind Inhalationsvorrichtungen bekannt, welche einen gasförmigen Brennstoff verbrennen. Aus der DE 19854009 A1 ist eine Inhalationsvorrichtung bekannt, bei der ein Heizmittel, insbesondere Propangas, in einer geschlossenen Brennkammer verbrannt wird. Aus der US 6,532,965 B1 sind Inhalationsvorrichtungen bekannt, bei denen Ethanol in einer Brennkammer verbrennt. Aus der US 6,598,607 B2 und der US 5,944,025 A sind Inhalationsvorrichtungen bekannt, bei denen Ethanol in einer katalytischen Reaktion ohne Flamme verbrennt.

Als nachteilig am Stand der Technik wird angesehen, dass die Handhabung der bekannten Inhalationsvorrichtungen sich deutlich von der einem Raucher bekannten üblichen Handhabung einer Zigarette unterscheidet, die Flamme in der Brennkammer nicht ausreichend stabil brennt und/oder die Stabilisierung des Verbrennungsprozesses aufwändige technische Maßnahmen erfordert.

### Aufgabe und Lösung

Aufgabe der Erfindung ist es, gattungsgemäße Heizeinrichtungen und Inhalationsvorrichtungen in Hinblick auf eine unkomplizierte Handhabung und zuverlässige Funktionsweise weiterzubilden.

Erfindungsgemäß wird dies durch eine gattungsgemäße Heizeinrichtung mit den kennzeichnenden Merkmalen des Anspruchs 1 erreicht.

In der Brennkammer der Heizeinrichtung wird der dorthin geförderte oder direkt dort gelagerte Brennstoff mit einer Flamme verbrannt. Vorzugsweise ist die Heizeinrichtung derart ausgebildet, dass nach Entzünden keine Energiezufuhr von außen zur Aufrechterhaltung der Flamme mehr erforderlich ist. Die erzeugte Wärme wird in einem der Heizeinrichtung zugeordneten Inhalationsgemischerzeuger genutzt, um ein zu inhalierendes Aerosol zu erzeugen. Die Nutzung der Wärme kann durch eine Erwärmung von Luft vor dem Eintreten in einen Inhalationsgemischerzeuger und/oder durch eine unmittelbare Erwärmung der zu inhalierenden Zusatzstoffe erfolgen.

Zur Integration der Heizeinrichtung in einer Inhalationsvorrichtung, die bezüglich ihrer Größe die Größe einer Zigarette nicht wesentlich übersteigt, ist die Heizeinrichtung in zwei der drei Dimensionen vorzugsweise kleiner als 11 mm.

Die Brennkammer weist einen freien Ausbreitungsbereich für die Flamme auf, der einen Durchmesser von mindestens 5 mm, vorzugsweise von mindestens 7 mm, aufweist. Vorzugsweise ist die Brennkammer zylindrisch ausgebildet und weist einen Durchmesser von mindestens 7 mm und eine Länge von mindestens 7 mm auf.

Als Brennkammer gilt der Raum, in dem sich die Flamme bei Gebrauch der Heizeinrichtung bestimmungsgemäß entfalten kann. Die Brennkammerwandung ist die Wandung, die die Brennkammer von einer Außenumgebung trennt, aus der der Sauerstoff zur Verbrennung stammt. Wandungen zu einem angrenzenden Inhalationsgemischerzeuger oder dem Brennstoffspeicher sind nicht Teil der Brennkammerwandung.

Die Angaben über die Randlängen der Mikrodurchbrechungen beziehen sich auf den Abschnitt der Brennkammerwandung, in dem die Mikrodurchbrechungen vorgesehen sind. Vorzugsweise sind die Mikrodurchbrechungen weitgehend homogen über die Brennkammerwandung verteilt. Sofern die Randlängendichte über die Brennkammerwandung variiert, beziehen sich die hier gemachten Angaben über die Randlängendichte auf einen Abschnitt der Brennkammerwandung, in dem sich bei relativ höchster Randlängendichte 80% der Mikrodurchbrechungen befinden.

Die Mikrodurchbrechungen, die in regelmäßiger, beispielsweise matrixartiger, oder in unregelmäßiger Anordnung vorgesehen sein können, gewährleisten eine kontinuierliche Sauerstoffversorgung der Flamme durch die Brennkammerwandung und ermöglichen dadurch eine Aufrechterhaltung der Flamme während der gesamten Nutzungsdauer. Bei einer besonderen Ausführungsform können die Mikrodurchbrechungen in Form von Schriftzeichen angeordnet sein, so dass Markenzeichen und ähnliches ohne Druckprozess aufgebracht werden können.

Die geringe Größe der Durchbrechungen schützt die Flamme vor äußeren Luftbewegungen und stabilisiert somit den Verbrennungsprozess. Die Ränder der Durchbrechungen erzeugen im Falle von Windstößen Wirbel in der Brennkammerwandung, welche die durchströmende Luft abbremsen. Aus aerodynamischer Sicht ist daher die Randlänge der Durchbrechungen maßgebend für den Strömungswiderstand der Brennkammerwandung. Bei gleicher offener Fläche führt eine höhere Randlänge pro Fläche zu einer Erhöhung des Strömungswiderstandes der Brennkammerwandung. Dadurch wird im Falle eines Windstoßes die Luftbewegung innerhalb der Brennkammer verringert, welche zu einem Erlöschen der Flamme führen könnte. Mikrodurchbrechungen mit hohen Randlängendichten ermöglichen daher im Gegensatz zu großen Durchbrechungen eine Stabilisierung der Flamme.

Als Rand der Mikrodurchbrechungen wird im Zusammenhang mit dieser Erfindung der Übergangsbereich zwischen Brennkammerwandung und Mikrodurchbrechung verstanden, wobei die entsprechenden Längenangaben auf ein optisches Auflösungsvermögen mit einer maximalen Auflösung von 5 Mikrometer bezogen sind. Die Längenangaben beziehen sich bei Mikrodurchbrechungen, die senkrecht zur Erstreckungsrichtung der Wandung verlaufen, auf eine plane Projektion. Bei Mikrodurchrechungen, die nicht senkrecht zur Erstreckungsrichtung der Wandung verlaufen, z.B. bei Verwendung einer Tresse, wird die absolute Filterfeinheit zur Bestimmung der Randlängen der Durchbrechungen herangezogen. Maßgebend für die Randlängenermittlung ist dementsprechend die maximale Randlänge eines Objektes, welches durch die jeweilige Mikrodurchbrechung passt.

Zur Zuführung des Brennstoffs in die Brennkammer kann ein Docht oder ein Druckrohr vorgesehen sein. Der Docht bzw. das Druckrohr können vorzugsweise mit einer oder mehreren der nachfolgenden Eigenschaften ausgebildet sein. Der Docht kann als Glasfaserdocht ausgebildet sein, der durch Kapillarkräfte den flüssigen Brennstoff in die Brennkammer fördert. Die Erweichungstemperatur des Dochts oder seines überwiegenden Teils kann über 800° C, vorzugsweise über 1000° C, liegen, um eine dünne Gestaltung des Dochts und damit einen geringen Sauerstoffbedarf der Flamme zu ermöglichen. Ein solcher Docht kann beispielsweise aus Quarzglas bestehen. Der Docht kann zur Vermeidung von Übergangsverlusten selbst den Brennstoffspeicher darstellen und zu diesem Zweck einen vorzugsweise Platz sparend zusammengelegten Brennstoffspeicherabschnitt aufweisen. Es können mehrere Dochte vorgesehen sein, die sich verschieden weit in die Brennkammer erstrecken, um eine Aufrechterhaltung der Flamme auch nach Erlöschen eines der Dochte oder eine stärkere Hitzeentwicklung durch mehrere Flammen bei starker Stauerstoffzufuhr zu ermöglichen.

Besondere Ausführungsformen sehen vor, dass im Bereich der Brennkammer mindestens ein dünnes Glühelement vorgesehen ist, welches durch die Flamme zum Glühen gebracht werden kann. Das Glühelement macht einen Betriebszustand der Heizeinrichtung sichtbar, was insbesondere bei Brennstoffen mit nicht sichtbarer Flamme zweckmäßig ist. Das Glühelement ist vorzugsweise als Chromnickeldraht ausgebildet.

Die Mikrodurchbrechungen sind vorzugsweise bezogen auf die Verwendung in einer zylindrischen Inhalationsvorrichtung an einer seitlichen Mantelwandung der Brennkammer vorgesehen. Vorzugsweise ist ein Bereich der Mikrodurchbrechungen in der Mantelwandung vorgesehen, der sich in Haupterstreckungsrichtung der Inhalationsvorrichtung über eine Länge zwischen 5 mm und 20 mm erstreckt.

Die Randlängendichte ist die Summe der Randlängen der Mikrodurchbrechungen pro Fläche. Die Randlängendichte wird ermittelt durch Bestimmung der Randlänge jeder einzelnen Durchbrechung, anschließende Aufsummierung über alle Durchbrechungen und Division durch die Fläche, auf der sich die Durchbrechungen verteilen.

Besonders vorteilhaft sind Mikrodurchbrechungen hinsichtlich des Einströmverhaltens der Luft und des Schutzes der Flamme, wenn die Randlängendichte mehr als 120 mm / cm², insbesondere mehr als 200 mm / cm² beträgt. Vorzugsweise beträgt die Randlängendichte über 400 mm / cm², insbesondere über 800 mm / cm². Die Gesamtrandlänge sollte mindestens bei 140 mm, insbesondere über 210 mm liegen. Vorzugsweise liegt die Gesamtrandlänge bei über 350 mm, vorzugsweise bei 700 mm, insbesondere über 1400 mm.

Als besonders bevorzugte Kombination hat sich eine Gesamtrandlänge von 140 mm bei einer Randlängendichte von 80 mm / cm² herausgestellt. Noch vorteilhafter sind 210 mm oder mehr Gesamtrandlänge bei 120 mm / cm² oder höherer Randlängendichte, beispielsweise 350 mm bei 200 mm / cm², 700 mm bei 400 mm / cm², 1400 mm bei 800 mm / cm². Eine höhere Randlängendichte und eine höhere Gesamtrandlänge werden jeweils als vorteilhaft angesehen. Weiterhin von Vorteil ist es dabei, wenn gegenüberliegende Ränder einer Mikrodurchbrechung maximal 1,4 mm voneinander beabstandet sind, um ein Hindurchdringen der Flamme zu vermeiden. Die Formgebung der Mikrodurchbrechungen kann verschieden geartet sein. Besonders einfach sind runde und quadratische Mikrodurchbrechungen herstellbar. Auch andere Formgebungen wie längliche, linienförmige oder schlangenlinienförmige Durchbrechungen sind geeignet.

Die Mikrodurchbrechungen selbst weisen bei einer Weiterbildung der Erfindung eine durchschnittliche Öffnungsfläche von weniger als 1,5 mm², vorzugsweise eine Öffnungsfläche von weniger als 1 mm², insbesondere vorzugsweise eine Öffnungsfläche von weniger als 0,5 mm² auf. Sie weisen darüber hinaus vorzugsweise eine Öffnungsfläche von mehr als 0,0002 mm², insbesondere mehr als 0,0004 mm² auf. Derartige Öffnungsflächen sind in Hinblick auf die Sauerstoffzufuhr und eine Vermeidung nachteiliger Luftstöße bis in die Brennkammer von Vorteil.

Der offene Bereich der Brennkammerwandung, der durch die Mikrodurchbrechungen geschaffen wird, nimmt vorzugsweise zwischen 10% und 50%, insbesondere zwischen 20% und 45% des Abschnitts der Brennkammerwandung ein, in dem die Mikrodurchbrechungen vorgesehen sind.

In einer Weiterbildung der Erfindung wird die Brennkammerwandung zumindest zum Teil durch ein Metallgewebe oder Metallgeflecht gebildet. Als vorteilhaft haben sich Metallgewebe und -geflechte mit einem Mesh-Wert zwischen 150 und 635, Metallgewebe und -geflechte mit einer Maschenweite zwischen 0,02 mm und 0,08 mm und/oder Metallgewebe und -geflechte mit einer Drahtstärke zwischen 0,019 mm und 0,05 mm herausgestellt.

Alternativ dazu kann auch die Ausbildung der Brennkammerwandung als Metallfolie mit eingebrachten Mikrodurchbrechungen von Vorteil sein. Die Mikrodurchbrechungen können dabei beispielsweise mechanisch oder mittels Ätzverfahren eingebracht werden.

Besonders bevorzugt sind Ausführungsformen, bei denen das Wandungsmaterial dünn ist und/oder eine relativ geringe Wärmekapazität aufweist. Dies führt dazu, dass die im Zuge des Entzündens eingebrachte Wärme nicht zur Erhitzung der Wandung abgeführt wird, sondern fast ausschließlich dem Entzünden dient. Eine leichtere Entzündbarkeit der Flamme in der Brennkammer ist die Folge. Bei Verwendung eines Metallgewebes oder einer Metallfolie als Wandungsmaterial, beispielsweise aus Chrom-Nickelstahl, sollte das Flächengewicht unter 300 Gramm/m² liegen.

In einer Weiterbildung der Erfindung ist die Brennkammer nur im Bereich mindestens und vorzugsweise eines Zündungszugangs von außen zugänglich. Die Zugänglichkeit ist dabei so zu verstehen, dass nur durch den Zündungszugang eine Anzündflamme beim Entzünden der Heizeinrichtung hindurchgelangen kann. Als Zündungszugang wird ein zusammenhängender Flächenbereich von mindestens 2 mm² angesehen, der zumindest zu 90% frei ist und einen offenen Kanal zwischen Umgebung und Brennkammer bildet. Vorzugsweise ist der Zündungszugang als länglicher Schlitz ausgebildet, der mindestens 1 mm breit ist. Der Zündungszugang ist bei einer bevorzugten Variante in einer seitlichen Wandung der Brennkammer vorgesehen. Alternativ kann der Zündungszugang auch am distalen Ende der Heizeinrichtung bezogen auf ihren mit einer Inhalationsvorrichtung verbundenen Zustand angeordnet sein. Vorzugsweise ist der Zündungszugang durch hintereinander angeordnete und/oder gegeneinander versetzte Flächenabschnitte so ausgebildet, dass die Luft beim Eindringen in die Brennkammer wenigstens einmal umgelenkt wird.

Als Brennstoff eignet sich insbesondere ein Ethanol-Wasser-Gemisch, da Ethanol eine nahezu vollständige Verbrennung ermöglicht, leicht entzündbar und nicht toxisch ist. Es können jedoch auch andere Brennstoffe Verwendung finden, wobei auch die Verwendung von toxischen Brennstoffen möglich ist, sofern die eingesogene Luft örtlich getrennt von der Brennkammer in die Vorrichtung eingesogen wird. Neben Ethanol haben sich andere Alkohole, Aldehyde, Ketone, Ester, n-Alkane mit einem bis vier Kohlenstoffatomen, n-Alkane mit fünf bis zwanzig Kohlenstoffatomen, verzweigte oder ringförmige Alkane mit vier bis zwanzig Kohlenstoffafomen, als zweckmäßig herausgestellt.

In einer Weiterbildung der Erfindung wird ein flüssiger Brennstoff mit einer Viskosität von mindestens 10.000 mPa·s verwendet. Die Flamme in der Brennkammer erhitzt die Oberfläche dieses Brennstoffgels über den Siedepunkt des Brennstoffs hinaus. Dadurch wird an der Oberfläche des Brennstoffgels Brennstoff verdampft und für den Erhalt der Flamme kontinuierlich bereitgestellt. Das Brennstoffgel gestattet eine einfache Handhabung und erlaubt darüber hinaus eine Anordnung unmittelbar in der Brennkammer und dadurch den Verzicht auf Dochte. Bei geringviskosen Brennstoffen kann durch den Zusatz von viskositätserhöhenden Additiven wie Polyacrylsäure oder Cellulose die gewünschte Viskosität erreicht werden.

Bei einer Weiterbildung der Erfindung ist der Brennstoffspeicher mittels eines gasdichten Verschlusses verschlossen, der durch Wärmezuführung entfernbar ist. Dadurch wird verhindert, dass sich der Brennstoff schon vor dem Entzünden verflüchtigt. Eine Entfernung des Verschlusses wird durch eine Wärmezuführung im Zuge der Entzündung der Flamme der Inhalationsvorrichtung erreicht. Als Materialien für den Verschluss kommen beispielsweise behandeltes Papier, Wachs, Zinnfolie oder Kunststofffolie in Betracht.

Alternativ hierzu kann vorgesehen sein, dass der Brennstoffspeicher mittels eines gasdichten Verschlusses verschlossen ist, der durch manuell aufbringbaren Druck auf den Brennstoffspeicher, vorzugsweise durch beidseitigen Druck auf die Heizeinrichtung im Bereich des Brennstoffspeichers, entfernbar oder aufreißbar ist. Ein solcher gasdichter Verschluss kann insbesondere eine Folie sein, die an einem Auslass eines ansonsten formstabilen Brennstofftanks vorgesehen ist. Der Benutzer kann die Heizeinrichtung bei einer Ausgestaltung gemäß dieser Weiterbildung durch einen leichten Druck in einen verwendbaren Zustand überführen.

Besonders vorteilhaft ist es, wenn eine Außenwandung des Brennstoffspeichers elastisch stark verformbar ist und durch eine Verschlussfolie abgeschlossen ist, die in geringerem Maße elastisch verformbar ist. Eine gemeinsame Verformung der Wandung und der Verschlussfolie führt dazu, dass die Verschlussfolie nach Erreichen der Reißgrenze aufreißt. Nach Wegfall der Verformungskraft kehrt der Brennstoffspeicher in seine ursprüngliche Form zurück und gestattet dann das Entweichen des Brennstoffs durch die gerissene Verschlussfolie.

Bei einer Weiterbildung der Erfindung ist der Brennstoffspeicher lösbar mit der Heizeinrichtung verbindbar und/oder wiederauffüllbar. Dies gestattet es, den Brennstoffspeicher wiederaufzufüllen oder austauschbar zu gestalten, wodurch andere Komponenten der Heizeinrichtung, insbesondere die Brennkammer mehrfach verwendet werden können.

Die Erfindung betrifft weiterhin eine gattungsgemäße Inhalationsvorrichtung, bei der die Heizeinrichtung nach oben beschriebener Art ausgebildet ist.

Durch das Mundstück, das beispielsweise als Hohlrohr oder Filter ausgebildet sein kann, kann der Benutzer Luft einsaugen, die dabei durch den Inhalationsgemischerzeuger geführt wird und dort mit mindestens einem Zusatzstoff vermengt wird. Das Gemisch wird nachfolgend vom Benutzer inhaliert.

Die Vermengung der Luft mit den Zusatzstoffen wird durch die hohe Temperatur der in der Brennkammer erhitzten Luft und/oder der erhitzten Zusatzstoffe erreicht. Es bildet sich dadurch ein inhalierbares Aerosol aus der eingesogenen Luft und den Zusatzstoffen. Durch die Wärme wird auch die Trennung der Zusatzstoffe von einer Trägerstruktur erreicht, in der die Zusatzstoffe gelagert sind. Die Wärme kann weiterhin eine chemische Modifikation der Zusatzstoffe und/oder der Trägerstruktur bewirken.

Die in der Brennkammer erzeugte Wärme wird vorzugsweise unmittelbar dem Inhalationsgemischerzeuger und/oder der eingesogenen Luft zugeführt, wobei die Flamme in der Brennkammer den Inhalationsgemischerzeuger und/oder die Luft unmittelbar oder mittelbar über wärmeleitende Materialien erwärmen kann. Um zu verhindern, dass die Flamme in der Brennkammer mit der Luft bis in den Inhalationsgemischerzeuger eingesogen wird, kann vor dem Inhalationsgemischerzeuger eine Flammenbarriere, beispielsweise aus Metallfolie, vorgesehen sein.

Die Zusatzstoffe sind vor der Vermengung mit der durchströmenden Luft vorzugsweise bereits in einem Zusatzstoffspeicher innerhalb des Inhalationsgemischerzeugers gelagert. Alternativ können sie in einem räumlich getrennten Zusatzstoffspeicher gelagert sein. Die Zusatzstoffe sind vorzugsweise in einem porösen Trägermaterial gelagert, wobei sich hierfür insbesondere Aktivkohle, Aluminiumoxid, Calciumkarbonat, Kieselgur, Zellulose oder Tabakmaterial anbieten. Zweckmäßig kann auch eine Lagerung der Zusatzstoffe in Pflanzenteilen sein, insbesondere in den Pflanzenteilen, die die Zusatzstoffe natürlich hervorbringen. Dies ist insbesondere bei Tabak mit Nikotin oder bei Heilkräutern mit Wirkstoffen zweckmäßig. Der Zusatzstoffspeicher kann durch eine Zigarette oder eine zumindest teilweise ummantelte oder anderweitig zusammengefügte Tabakeinheit gebildet werden. Bei Inhalationsgemischerzeugern mit mehreren Zusatzstoffen sind diese vorzugsweise hintereinander angeordnet, wobei hitzeempfindlichere Zusatzstoffe in einem hinteren Bereich des Strömungswegs der Luft angeordnet sind.

Die Brennkammer ist vorzugsweise so ausgebildet und in der Inhalationsvorrichtung angeordnet, dass die Flamme von außen sichtbar ist. Sie ist vorzugsweise derart zugänglich, dass ein Entzünden der Flamme mit den herkömmlicherweise bei Zigaretten üblichen Mitteln wie Streichhölzern oder Feuerzeugen von außen möglich ist.

Die Komponenten der Inhalationsvorrichtung sind vorzugsweise in einem einheitlichen Gehäuse mit einer gemeinsamen Außenwandung angeordnet. Dabei ist es besonders vorteilhaft, wenn die Formgebung des Gehäuses an übliche Tabakwaren wie an eine Zigarette angenähert ist. Bevorzugt werden Ausführungsformen, bei denen die Außenwandung zumindest zum Teil aus nicht oder schwer entflammbarem Material besteht, beispielsweise aus vorbehandeltem Papier, aus Metall, aus Keramik oder aus Porzellan.

Der Haupteinsatzzweck erfindungsgemäßer Inhalationsvorrichtungen ist der Ersatz üblicher Tabakwaren, bei denen die Wirkstoffe und Zusatzstoffe durch eine Verbrennung von Tabak freigesetzt werden. Bei einer erfindungsgemäßen Inhalationsvorrichtung können die Zusatzstoffe unter Vermeidung krankheitserzeugender, insbesondere krebserzeugender Zusatzstoffe und Verbrennungsprodukte sehr gezielt ausgewählt werden. So bieten sich für einen Zigarettenersatz insbesondere Zusatzstoffkombination mit dem Wirkstoff Nikotin und Aromastoffen an. Als geschmacksbildende Zusatzstoffe sind insbesondere Öle, Tabakpflanzenextrakte sowie natürliche und naturidentische Aromastoffe zweckmäßig. Darüber hinaus sind zur Nachahmung herkömmlicher Zigaretten auch vorzugsweise geschmacksneutrale nebelerzeugende Substanzen wie Polyole, insbesondere Propylenglycol oder Glycerol, zweckmäßig. Es kann aber auch herkömmlicher Tabak Verwendung finden, dessen Schädlichkeit durch die Vermeidung einer Verbrennung des Tabaks erheblich gesenkt wird.

Neben der Verwendung als Ersatz herkömmlicher Tabakwaren sind auch andere Anwendungen möglich, beispielsweise medizinische Anwendungen, bei denen der Zusatzstoff eine insbesondere pharmazeutische Wirkung hat. Dies umfasst zum Beispiel Schmerzmittel und Beruhigungsmittel.

Bei einer Weiterbildung ist die Heizeinrichtung als separat handhabbare Vorrichtung ausgebildet, die mit dem Inhalationsgemischerzeuger lösbar verbindbar ist. Dies gestattet es, die Heizeinrichtung wiederzuverwenden, während der Inhalationsgemischerzeuger, vorzugsweise gemeinsam mit dem Mundstück und einem Zusatzstoffspeicher, als Einweg-Komponente gehandhabt wird. Insbesondere können die Komponenten Mundstück, Inhalationsgemischerzeuger und Zusatzstoffspeicher auch gemeinsam durch eine handelsübliche Zigarette oder eine abgepackte Tabakeinheit mit Mundstück gebildet sein, an der die Heizeinrichtung derart anbringbar ist, dass die durch die Zigarette oder die Tabakeinheit eingesogenen Luft mittels der Flamme in der Brennkammer der Heizeinrichtung erhitzt wird. Eine solche Heizeinrichtung wird vorzugsweise am distalen, also dem Benutzer abgewandten Ende, auf die Zigarette oder die Tabakeinheit aufgesetzt.

Zweck dieser Kombination aus Heizeinrichtung und derartigem Tabakerzeugnis ist es, dem Tabakerzeugnis Wärme aus der Heizeinrichtung zuzuführen, um eine Trennung der Wirkstoffe und/oder Geschmackstoffe von dem Tabak zu erreichen. Die in der Brennkammer erzeugte Wärme wird durch Konvektion und/oder Wärmeleitung dem Tabak zugeführt und erwärmt diesen auf 150°C bis 400°C. Dadurch werden die Zusatzstoffe aus dem Tabak gelöst, ohne dass es zu einer Entzündung des Tabakerzeugnisses kommt. Der Benutzer kann sein gewohntes Tabakerzeugnis weiterverwenden, wobei dessen schädigende Eigenschaften verringert werden. Im Falle einer derartigen Heizeinrichtung, die für die Verwendung mit einer normalen Zigarette geeignet ist, wäre der Aufsatz vorzugsweise so auszubilden, dass er den Abschnitt der Zigarette, der mit Tabak befüllt ist, vollständig oder weitgehend umschließt. Denkbar sind auch Ausführungsformen, bei denen auch das Mundstück Teil des Aufsatzes ist, so dass von der Zigarette lediglich der Tabak genutzt wird. Bei einer Weiterbildung der Erfindung lässt sich die Heizeinrichtung auf die Lufteinlassöffnung einer Pfeife aufsetzen.

Bei einer Weiterbildung der Inhalationsvorrichtung ist ein vom Inhalationsgemischerzeuger getrennt ausgebildeter Zusatzstoffspeicher vorgesehen, wobei der Zusatzstoffspeicher austauschbar und/oder wiederbefüllbar ausgebildet ist. Die Austauschbarkeit des Zusatzstoffspeichers ermöglicht es, bestimmte Bestandteile, wie beispielsweise das Gehäuse, die Heizeinrichtung und/oder das Mundstück wiederholt zu verwenden. Der Zusatzstoffspeicher kann an die Inhalationsvorrichtung in einfacher Art und Weise angesteckt oder in die Inhalationsvorrichtung eingesetzt werden.

Bei einer Weiterbildung der Erfindung weist die Inhalationsvorrichtung eine im Wesentlichen längliche Struktur, vorzugsweise eine zylindrische Struktur auf, wobei das Mundstück an einem proximalen Ende der Inhalationsvorrichtung angeordnet ist und wobei die Heizeinrichtung im Bereich des distalen Endes der Inhalationsvorrichtung angeordnet ist. Eine solche Inhalationsvorrichtung kommt bezüglich der Formgebung einer Zigarette bzw. Zigarre besonders nahe und bildet somit einen guten Zigarettenersatz.

### Kurzbeschreibung der Zeichnungen

Weitere Vorteile und Merkmale der Erfindung ergeben sich aus den Ansprüchen sowie der nachfolgenden Beschreibung von bevorzugten Ausführungsbeispielen der Erfindung, die anhand der Zeichnungen dargestellt ist. Die Ausführungsbeispiele dienen nur der Erläuterung und schränken die Erfindung nicht ein. Es zeigt:
- Fig. 1 a bis 1 c: eine erste Ausführungsform einer erfindungsgemäßen Inhalationsvorrichtung in einer ungeschnittenen und einer geschnittenen Ansicht sowie in einem Betriebs- zustand,
- Fig. 2a bis 2c: eine zweite Ausführungsform einer erfindungsgemä- ßen Inhalationsvorrichtung in einer ungeschnittenen und einer geschnittenen Ansicht sowie in einem Be- triebszustand,
- Fig. 3a bis 3c: eine dritte Ausführungsform einer erfindungsgemäßen Inhalationsvorrichtung in einer ungeschnittenen und einer geschnittenen Ansicht sowie in einem Betriebs- zustand,
- Fig. 4a bis 4c: eine vierte Ausführungsform einer erfindungsgemä- ßen Inhalationsvorrichtung in einer ungeschnittenen und einer geschnittenen Ansicht sowie in einem Be- triebszustand und
- Fig. 5a bis 5c: verschiedene Brennkammerwandungen im abgeroll- ten Zustand.

### Detaillierte Beschreibung der Ausführungsbeispiele

Im Zusammenhang mit der Erfindung wird unter dem proximalen Ende stets das dem Benutzer zugewandte Ende einer Komponente verstanden und unter dem distalen Ende das vom Benutzer weggewandte. Bezüglich ihrer Funktion vergleichbare Komponenten der verschiedenen Ausführungsbeispiele stimmen in den letzten beiden Ziffern des Bezugszeichens überein.

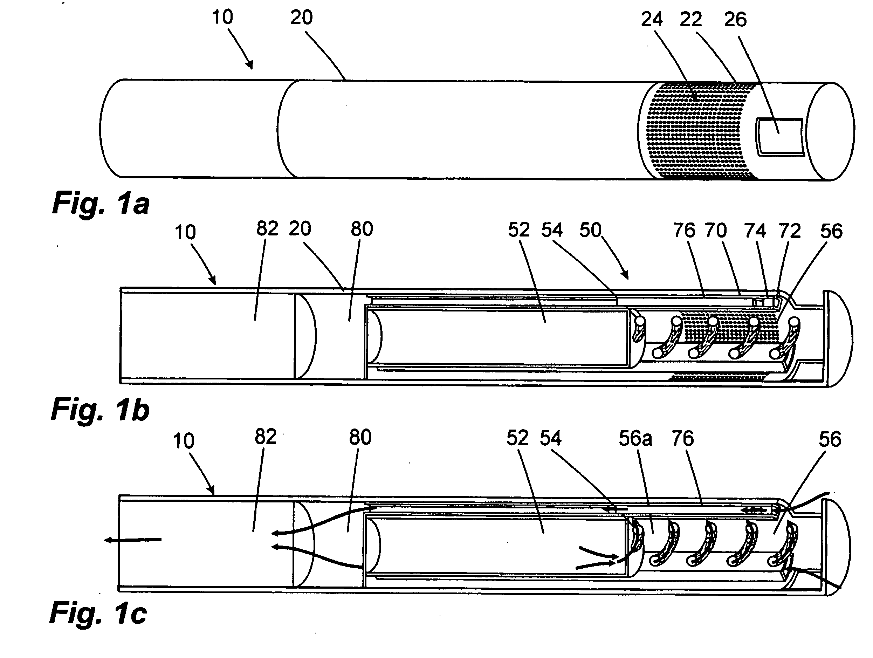

Die Figuren 1a bis 1c zeigen eine erste Ausführungsform einer erfindungsgemäßen Inhalationsvorrichtung 10. Diese ist zylinderförmig ausgebildet und weist eine Außenwandung 20 auf, die an einem distalen Ende der Inhalationsvorrichtung 10 als Brennkammerwandung 22 mit Mikrodurchbrechungen 24 versehen ist. An der distalen Stirnseite weist die Wandung 20 eine Aussparungen 26 auf.

Figur 1b zeigt den inneren Aufbau der Inhalationsvorrichtung 10. Am distalen Ende der Inhalationsvorrichtung 10 ist eine Heizeinrichtung 50 vorgesehen. Die Heizeinrichtung weist einen Brennstoffspeicher 52 auf, in dem der Brennstoff flüssig gelagert ist. Aus dem Brennstoffspeicher 52 erstreckt sich ein Docht 54 in eine Brennkammer 56, die am distalen Ende der Inhalationsvorrichtung vorgesehen ist und die von der Brennkammerwandung 22 umgeben wird. Der Docht 54 ist schraubenartig geformt. An einer Innenseite der Außenwandung 20 sind über den Umfang verteilt insgesamt drei Inhalationsgemischerzeuger 70 angeordnet, die sich jeweils im Wesentlichen axial vom distalen Ende der Inhalationsvorrichtung ausgehend in Richtung des proximalen Endes erstrecken. Die Inhalationsgemischerzeuger 70 sind als Hohlkanäle ausgebildet, in deren Lufteinlassöffnungen 72 als Flammenbarriere wirkende Glasfaserpfropfen 74 eingefügt sind. In den röhrenförmigen Inhalationsgemischerzeugern 70 sind Zusatzstoffe 76 in einer porösen Trägersubstanz gelagert. An einem proximalen Ende der Inhalationsgemischerzeuger 70 schließt sich an diese eine Homogenisierungskammer 80 an, in die alle drei Inhalationsgemischerzeuger 70 münden. Das proximale Ende der Inhalationsvorrichtung 10 wird durch ein Mundstück 82 mit eingesetztem Filter gebildet, der den Querschnitt der Außenwandung 20 vollständig ausfüllt.

Die Figur 1c verdeutlicht die Funktionsweise der Inhalationsvorrichtung 10. Die Inhalationsvorrichtung wird mit ihrem proximalen Ende in der Art einer Zigarette in den Mund gesteckt. Sie wird dann entzündet, indem eine externe Flamme, beispielsweise von einem Feuerzeug oder einem Streichholz, an das distale Ende der Inhalationsvorrichtung 10 gehalten wird. Dadurch entzündet sich der Docht 54, der aus dem Brennstoffspeicher 52 mit einem Brennstoff, beispielsweise mit einem Alkohol versorgt wird. In der Brennkammer 56 bildet sich dadurch eine Flamme. Der Benutzer kann Luft durch das Mundstück 82 in die Inhalationsgemischerzeuger 70 einsaugen. Die eingesogene Luft durchströmt die Zusatzstoffe 76, die in dem porösen Trägermaterial gespeichert sind. Durch die Erwärmung werden die Zusatzstoffe 76 an die einströmende Luft abgegeben und vermengen sich mit dieser zu einem Inhalationsgemisch. Dieses Inhalationsgemisch wird durch die Homogenisierungskammer 80 und das Mundstück 82 gesogen.

Durch die Mikrodurchbrechungen 24 in der Außenwandung 20 im Bereich der Brennkammer 56 wird eine hohe Stabilität der Flamme erreicht. Bei Fehlen einer Beeinträchtigung, beispielsweise durch Wind, ist die Flamme als laminare Flamme im Bereich der Stirnseite der Inhalationsvorrichtung ausgebildet. Im Falle, dass es am distalen Ende zu einer starken Luftströmung, beispielsweise durch Wind oder durch eine schnelle Bewegung der Inhalationsvorrichtung 10 kommt, kann eine Abreißen der laminaren Flamme die Folge sein. In einem solchen Fall kann sich die Flamme in einen windgeschützteren hinteren Bereich 56a der Brennkammer 56 zurückziehen, wobei die Luftzuführung dann immer noch über die Mikrodurchbrechungen 24 gewährleistet ist.

Die dargestellte und beschriebene Inhalationsvorrichtung 10 weist gegenüber einer normalen Zigarette deutliche Vorteile in Hinblick auf eine Gesundheitsbelastung des Benutzers auf. Während bei einer normalen Zigarette eine Vielzahl an unerwünschten Zusatzstoffen mitinhaliert werden, die insbesondere durch die Verbrennung des Tabaks freigesetzt werden, werden bei der dargestellten Inhalationsvorrichtung 10 lediglich die Zusatzstoffe 76 inhaliert, die insbesondere frei sind von krebserzeugenden Verbrennungsprodukten. Je nach Wahl der Zusatzstoffe 76 kann dennoch ein ähnlicher Geschmack sowie eine ähnliche Wirkung in Hinblick auf das in Zigaretten enthaltene Nikotin erzielt werden.

Die Verwendung der Inhalationsvorrichtung stellt verglichen mit dem Rauchen einer Zigarette keine wesentlichen Änderungen dar. Wie eine Zigarette wird die Inhalationsvorrichtung an ihrem distalen Ende entzündet und bleibt dann im entzündeten Zustand, bis die Zusatzstoffe 76 aufgebraucht sind oder die Flamme zum Erlöschen gebracht wird. Es kann auch zweckmäßig sein, die Brennstoffmenge im Brennstoffspeicher 52 derart zu begrenzen, dass der Brennstoff in etwa gleichzeitig mit den Zusatzstoffen 76 aufgebraucht ist.

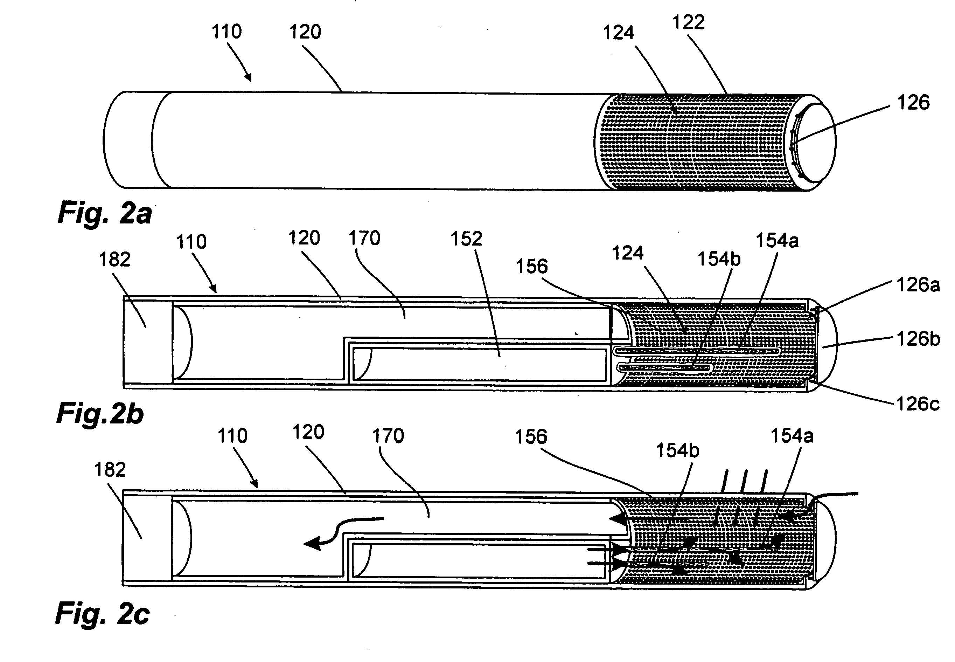

Die Figuren 2a bis 2c zeigen eine zweite Ausführungsform einer erfindungsgemäßen Inhalationsvorrichtung. Diese Inhalationsvorrichtung 110 ist in vielerlei Hinsicht der Ausführungsform der Fig. 1 ähnlich. Soweit im Folgenden nichts anderes angegeben ist, ist ihre Funktionsweise die gleiche.

Die Inhalationsvorrichtung 110 weist eine Außenwandung 120 auf, deren vorderes Ende als Brennkammerwandung 122 ausgebildet ist. Innerhalb dieser Außenwandung 120 sind ein Brennstoffspeicher 152, eine Brennkammer 156 sowie ein Inhalationsgemischerzeuger 170 vorgesehen.

Die Anordnung des Inhalationsgemischerzeugers 170 und des Brennstoffspeichers 152 weicht von den Ausführungsformen der Fig. 1 ab. Während der Inhalationsgemischerzeuger 170 im hinteren Bereich der Inhalationsvorrichtung 110 den vollständigen Querschnitt einnimmt, teilt sich der Querschnitt im mittleren Bereich des Inhalationsgemischerzeugers 170 in zwei halbkreisförmigen Querschnittsbereiche auf, wobei der eine vom Brennstoffspeicher 152 und der andere vom Inhalationsgemischerzeuger 170 eingenommen wird. Dieser vereinfachte Aufbau ist in Hinblick auf die Herstellungskosten von Vorteil. Bei der dargestellten Ausführungsform weist der Inhalationsgemischerzeuger 170 eine Außenwandung auf. Je nach Ausgestaltung der Außenwandung 120 der Inhalationsvorrichtung 110 kann auf eine solche zusätzliche Wandung jedoch verzichtet werden. Der Inhalationsgemischerzeuger 170 ist bei dieser Ausführungsform vergleichsweise groß ausgeführt und kann dadurch größere Mengen an Geschmacksstoffen, Tabak oder ähnlichem aufnehmen.

Wesentliche Unterschiede bestehen in Hinblick auf die Brennkammer 156. Bei der Ausführungsform der Fig. 2a bis 2c ist diese größtenteils durch den Abschnitt 122 der Außenwandung 120 umgeben, der mit einer Vielzahl von Mikrodurchbrechungen 124 versehen ist. Am distalen Ende der Inhalationsvorrichtung 110 weist die Außenwandung 120 eine Zugangsöffnung 126 auf. Diese wird durch eine Durchbrechung 126a gebildet, die durch eine Windschutzfläche 126b derartig verdeckt ist, dass der Luftzugang nur durch einen dazwischen liegende umlaufenden Spalt 126c möglich ist. Die Brennkammer 156 ist durch die Zugangsöffnung 126 demzufolge nur mittelbar von außen zugänglich. Diese Ausgestaltung erlaubt ein Entzünden der Flamme in der Brennkammer mittels Streichhölzern oder Feuerzeug, während an der Inhalationsvorrichtung 110 gezogen wird. Durch den nur schmalen Spalt 126c und die Tatsache, dass einströmende Luft zwangsläufig umgelenkt werden muss, um von außen bis in den Bereich der Flamme zu gelangen, ist gewährleistet, dass die Flamme in der Brennkammer nicht durch einen Windstoß zum Erlöschen gebracht werden kann.

Innerhalb der Brennkammer 156 sind mehrere Dochte 154a, 154b aus Quarzglas vorgesehen, von denen lediglich ein langer innerer Docht 154a und ein kurzer äußerer Docht 154b dargestellt sind. Die Dochte bestehen jeweils aus Quarzglasfilamenten mit einem Durchmesser von 12,5 Mikrometer, wobei in jedem Docht etwa 100 bis 200 Filamente verarbeitet sind. Aber auch Fasern mit einem Durchmesser von beispielsweise 50 bis 100 Mikrometer können gut verwendet werden. Im ungezündeten Zustand der Fig. 2b sind die Dochte durch eine Schutzfolie umgeben, die ein Austreten des Brennstoffes aus der Brennkammer verhindert. Bei einer nicht dargestellten Ausführungsform entfallen die Dochte. Stattdessen liegt der Brennstoff in gelartigem Zustand vor.

Die Inhalationsvorrichtung wird durch ein Feuerzeug oder ein Streichholz gezündet, welches im Bereich des Spalts 126c der Zugangsöffnung 126 an die Inhalationsvorrichtung 110 herangehalten wird. Durch Einsaugen von Luft wird die Flamme des Feuerzeugs oder Streichholzes bis in den Bereich des längeren Dochtes 154a eingesogen, wo sie zur Entzündung des längeren Dochtes 154a führt. Durch die Verwendung von Quarzglas als Material des Dochtes können aufgrund des hohen Schmelzpunktes besonders feine Dochte 154a, 154b verwendet werden. Diese führen zu kleinen Flammen mit geringem Sauerstoffbedarf.

Im Betrieb brennt die Flamme zumeist am vorderen Ende des längeren Dochts 154a, da die Sauerstoffzuführung dort am besten gewährleistet ist. Sobald der Benutzer kräftig am Mundstück 182 zieht, wird allerdings in erhöhtem Maße Luft durch die Mikrodurchbrechungen 124 in die Brennkammer 156 eingesogen. Dies führt zu einer Ausbreitung der Flamme am Docht 154a in Richtung zum proximalen Ende des Dochts 154a hin. Hierbei kommt es zur Entzündung des kurzen Dochtes 154b sowie ggf. weiterer ähnlich angeordneter Dochte. Dies führt zu einer Erhöhung der Wärmeabgabe an die Luft, so dass auch bei starkem Inhalieren durch einen Benutzer die notwendige Wärme durch die Flammen in der Brennkammer bereitgestellt werden kann.

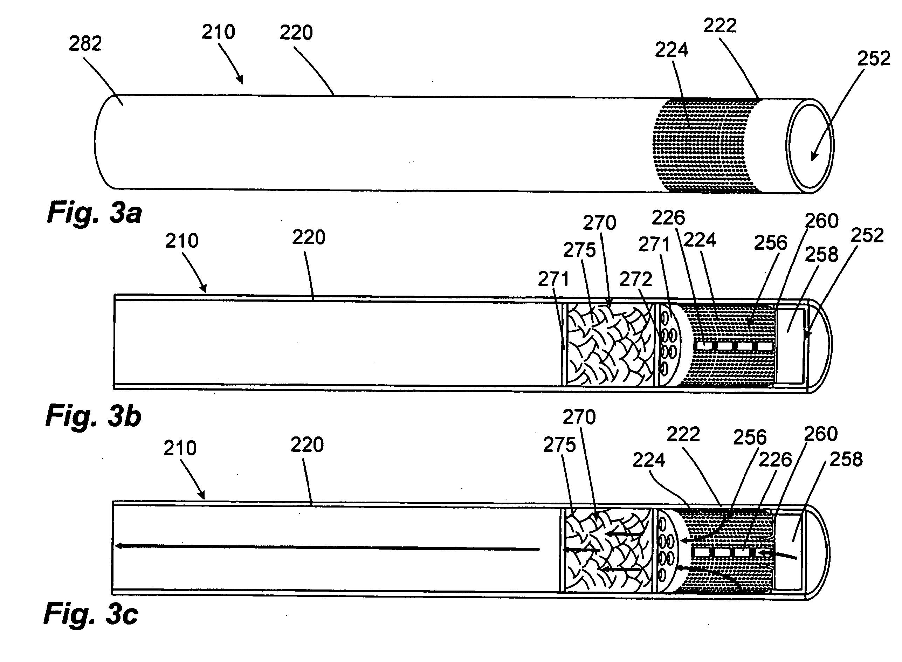

Eine dritte Ausführungsform ist den Fig. 3a bis 3c zu entnehmen. Die dargestellte Inhalationsvorrichtung 210 verfügt über eine zylindrische Form mit einer mantelförmigen Außenwandung 220. Diese ist am distalen Ende durch einen napfförmigen Brennstoffspeicher 252 aus Chromnickel-Federstahl abgeschlossen, wobei der Brennstoffspeicher 252 einen Brennstoff 258 in einer gelartigen Konsistenz enthält und in Richtung einer Brennkammer 256 offen ist. Vor der Inbetriebnahme der Inhalationsvorrichtung 210 ist, wie in Fig. 3b ersichtlich, die offene Seite des Brennstoffspeichers 252 durch eine Membran 260 aus einem Material mit geringer Elastizität, beispielsweise durch Aluminiumfolie, abgeschlossen.

Die Brennkammer 256 ist von Mikrodurchbrechungen 224 in einem Brennkammerwandungsabschnitt 222 der Außenwandung 220 umgeben. An einer Seite ist zusätzlich ein Zündungszugang 226 vorgesehen, der aus insgesamt vier länglichen Öffnungen besteht, zwischen denen lediglich schmale Stege vorgesehen sind. An der proximalen Seite schließt sich an die Brennkammer 256 ein Inhalationsgemischerzeuger 270 an. Dieser ist als mit einer Mischung 275 aus Tabak und Tabakextrakt gefüllte Kammer ausgebildet, die brennkammerseitig und benutzerseitig durch Wandungen 271 mit Eingangsöffnungen 272 bzw. nicht dargestellten Ausgangsöffnungen abgeschlossen ist.

Zur Benutzung der Inhalationsvorrichtung 210 wird zunächst der Brennstoffspeicher 252 von außen leicht zusammengedrückt. Er verformt sich dadurch elastisch, während die Membran 260 aufgrund ihrer geringen Elastizität reißt. Der Brennstoff 258 wird dadurch brennkammerseitig zugänglich, wie in Fig. 3c dargestellt ist. Anschließend wird der gelartige Brennstoff 258 mittels einer von außen an die Zugangsöffnung 226 gehaltene Flamme entzündet, so dass sich in der Brennkammer 256 eine Flamme bildet, die von Brennstoff 258 gespeist wird. Diese Flamme ist aufgrund der Abschottung der Brennkammer 256 sehr stabil. Durch die Mikrodurchbrechungen 224 wird sie kontinuierlich zuverlässig mit Sauerstoff versorgt, ohne dass ein plötzlicher Windstoß oder ähnliches sie zum Erlöschen bringen kann.

Der Benutzer, der am Mundstück 282 zieht, zieht dadurch die auf Temperaturen von über 250°C erhitzte Luft aus der Brennkammer 256 in den Inhalationsgemischerzeuger 270. Dort durchströmt die heiße Luft die Mischung 275 und löst dabei die Zusatzstoffe, die anschließend vom Benutzer inhaliert werden. Der Rauchvorgang ist zeitlich durch die Menge des Brennstoffs 258 und durch die Menge der Zusatzstoffe in der Mischung 275 begrenzt.

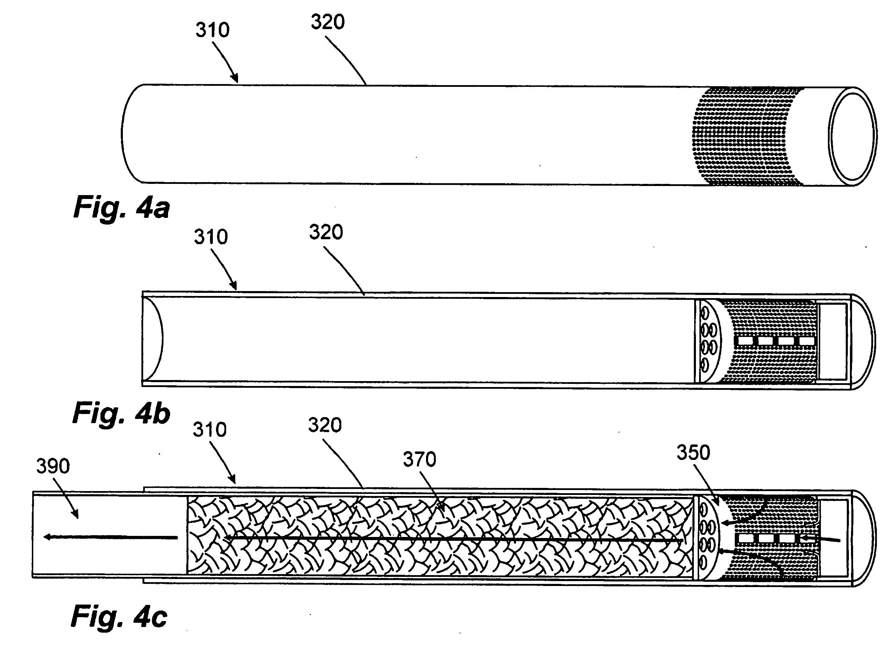

Die Ausführungsform der Fig. 4a bis 4c entspricht weitgehend der Ausführungsform der Fig. 3a bis 3c. Der einzige Unterschied liegt im Inhalationsgemischerzeuger 370. Dieser wird bei der Ausführungsform der Fig. 4a bis 4c durch eine handelsübliche Zigarette 390 gebildet, welche in die Außenhülse 320 der Inhalationsvorrichtung 310 eingeschoben ist. Die Inhalationsvorrichtung besteht bei dieser Ausführungsform im Kern lediglich aus der Heizeinrichtung 350.

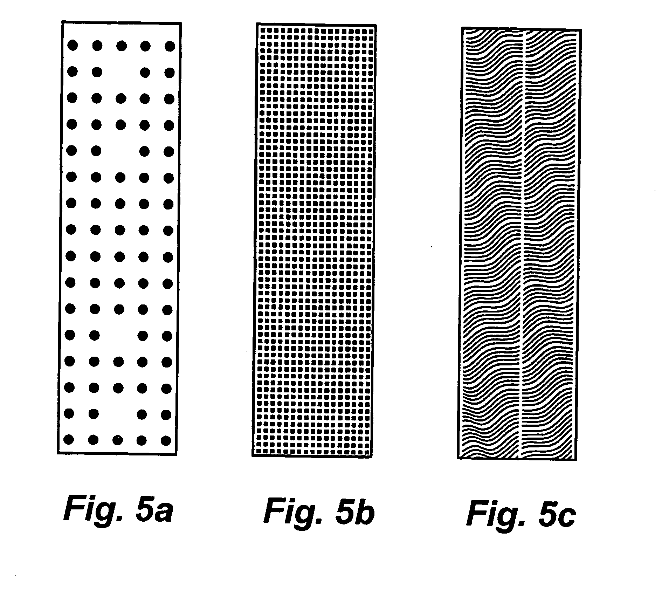

Die Fig. 5a bis 5c zeigen verschiedene Brennkammerwandungen im abgerollten Zustand. Diese Brennkammerwandungen können als separate Bauteile bei den vorbeschriebenen und anderen Inhalationsvorrichtungen verarbeitet werden oder Teil von größeren Außenwandungen sein, die nur abschnittsweise die Brennkammerwandung bilden. Bei den dargestellten Wandungsabschnitten handelt es sich jeweils um 7 mm breite und 25 mm lange Streifen, die im fertig gestellten Zustand eine zylindrische Brennkammerwandung von 7 mm Länge und etwa 8 mm Durchmesser bietet. Die Gesamtfläche der Brennkammerwandungen beträgt 1,75 cm².

Bei der Ausführungsform der Fig. 5a sind dabei insgesamt 75 runde Mikrodurchbrechungen auf dieser Fläche vorgesehen, die jeweils eine Randlänge von etwa 2 mm aufweisen. Die Gesamtrandlänge beträgt daher etwa 140 mm. Bezogen auf die Fläche beträgt die Randlänge etwa 80 mm / cm². Diese Mikrodurchbrechungswerte in Hinblick auf die Randlängen werden als Mindestwerte angesehen.

Bei der Ausführungsform der Fig. 5b handelt es sich um insgesamt 1093 Durchbrechungen, die jeweils eine Randlänge von 1 mm aufweisen. Die Gesamtrandlänge beträgt daher etwa 1093 mm und die auf die Fläche bezogene Randlänge beträgt etwa 625 mm / cm^{2.}

Bei der Ausführungsform der Fig. 5c handelt es sich um insgesamt etwa 150 linienförmige Durchbrechungen, die jeweils eine Randlänge von etwa 9 mm aufweisen. Die Gesamtrandlänge beträgt daher etwa 1350 mm und die auf die Fläche bezogene Randlänge beträgt etwa 700 mm / cm^{2.}

Die Ausführungsformen der Fig. 5b und 5c werden als besonders vorteilhaft angesehen.

## Patentansprüche

1. Inhalationsvorrichtung (10; 110; 210; 310) zur inhalativen Einnahme eines Inhalationsgemisches aus Luft und mindestens einem Zusatzstoff mit
- einem Mundstück (82; 182; 282),
- einem Inhalationsgemischerzeuger (70; 170; 270; 370) mit einer Lufteinlassöffnung (72; 272),
- einer Heizeinrichtung (50; 350) zur Verbrennung eines Brennstoffs (258) und zur Erhitzung der Luft und/oder des Zusatzstoffs und/oder des Inhalationsgemisches,
wobei
- der Inhalationsgemischerzeuger (70; 170; 270; 370) mit dem Mundstück (82; 182; 282) unmittelbar oder über einen Inhalationskanal verbunden ist,
**dadurch gekennzeichnet, dass**
die Heizeinrichtung (50; 350)
- einen vom Inhalationsgemischerzeuger räumlich getrennten Brennstoffspeicher (52; 152; 252) aufweist, der mit einem thermisch brennbaren festen oder flüssigen Brennstoff (258) befüllbar oder befüllt ist, und
- eine zur Ausbildung einer Flamme ausgebildete Brennkammer (56; 156; 256) zur Verbrennung des Brennstoffs (258) aufweist, die von der Umgebung durch eine Brennkammerwandung (22; 122; 222), die eine Außenwandung der Inhalationsvorrichtung bildet, weitgehend abgeschlossen ist, wobei
- die Brennkammerwandung (22; 122; 222) zumindest zum Teil Mikrodurchbrechungen (24; 124; 224) aufweist, die so ausgebildet sind, dass
- die Summe der Randlängen aller Mikrodurchbrechungen (24; 124; 224) mindestens 140 mm beträgt und
- die Summe der Randlängen der Mikrodurchbrechungen (24; 124; 224) pro Fläche im Bereich der Brennkammerwandung (22; 122; 222) mindestens 80 mm / cm² im Mittel beträgt.

2. Inhalationsvorrichtung nach Anspruch 1,
**dadurch gekennzeichnet, dass**
die Mikrodurchbrechungen (24; 124; 224) mehrheitlich an einer bezogen auf eine Haupterstreckungsrichtung der Inhalationsvorrichtung seitlichen Wandung (20; 120; 220; 320) vorgesehen sind.

3. Inhalationsvorrichtung nach einem der Ansprüche 1 oder 2,
**dadurch gekennzeichnet, dass**
- die Summe der Randlängen aller Mikrodurchbrechungen (24; 124; 224) mindestens 700 mm beträgt und
- die Summe der Randlängen der Mikrodurchbrechungen (24; 124; 224) pro Fläche im Bereich der Brennkammerwandung (22; 122; 222) mindestens 400 mm / cm² im Mittel beträgt.

4. Inhalationsvorrichtung nach einem der vorstehenden Ansprüche, **dadurch gekennzeichnet, dass**
die Mikrodurchbrechungen (24; 124; 224) eine durchschnittliche Öffnungsfläche von weniger als 1,5 mm² aufweisen.

5. Inhalationsvorrichtung nach einem der vorstehenden Ansprüche, **dadurch gekennzeichnet, dass**
ein durch die Mikrodurchbrechungen (24; 124; 224) offener Bereich der Brennkammerwandung (22; 122; 222) zwischen 10% und 50% eines mit den Mikrodurchbrechungen (24; 124; 224) versehenen Abschnitts der Brennkammerwandung (22; 122; 222) einnimmt.

6. Inhalationsvorrichtung nach einem der vorstehenden Ansprüche, **dadurch gekennzeichnet, dass**
die Brennkammerwandung zumindest zum Teil durch ein Metallgewebe oder Metallgeflecht aus Metalldrähten gebildet wird.

7. Inhalationsvorrichtung nach einem der vorstehenden Ansprüche, **dadurch gekennzeichnet, dass**
die Brennkammerwandung (22; 122; 222) zumindest zum Teil durch eine Metallfolie mit eingebrachten Mikrodurchbrechungen (24; 124; 224) gebildet wird.

8. Inhalationsvorrichtung nach einem der vorstehenden Ansprüche, **dadurch gekennzeichnet, dass**
die Brennkammer (56; 156; 256) nur im Bereich eines Zündungszugangs (26; 126; 236) von außen zugänglich ist.

9. Inhalationsvorrichtung nach einem der vorstehenden Ansprüche, **dadurch gekennzeichnet, dass**
der Brennstoff (258) zumindest zum Teil aus einem oder mehreren der aus der folgenden Gruppe ausgewählten Substanzen besteht: Alkohole, Aldehyde, Ketone, Ester, n-Alkane mit einem bis vier Kohlenstoffatomen, n-Alkane mit fünf bis zwanzig Kohlenstoffatomen, verzweigte oder ringförmige Alkane mit vier bis zwanzig Kohlenstoffatomen.

10. Inhalationsvorrichtung nach einem der vorstehenden Ansprüche, **dadurch gekennzeichnet, dass**
der Brennstoffspeicher (152) mittels eines gasdichten Verschlusses verschlossen ist, der durch Erhitzung entfernbar ist.

11. Inhalationsvorrichtung nach einem der vorstehenden Ansprüche, **dadurch gekennzeichnet, dass**
der Brennstoffspeicher (252) mittels eines gasdichten Verschlusses (260) verschlossen ist, der durch manuell aufbringbaren Druck auf den Brennstoffspeicher, vorzugsweise durch beidseitigen Druck auf die Inhalationsvorrichtung im Bereich des Brennstoffspeichers, entfernbar oder aufreißbar ist.

12. Inhalationsvorrichtung nach einem der vorstehenden Ansprüche, **dadurch gekennzeichnet, dass**
der Brennstoffspeicher (252) lösbar mit der Heizeinrichtung verbindbar ist.

13. Inhalationsvorrichtung nach einem der vorstehenden Ansprüche, **dadurch gekennzeichnet, dass**
die Heizeinrichtung (350) als separat handhabbare Vorrichtung ausgebildet ist, die mit dem Inhalationsgemischerzeuger (370) lösbar verbindbar ist.

14. Inhalationsvorrichtung nach einem der vorstehenden Ansprüche, **dadurch gekennzeichnet, dass**
ein vom Inhalationsgemischerzeuger getrennt ausgebildeter Zusatzstoffspeicher vorgesehen ist, wobei der Zusatzstoffspeicher austauschbar oder wiederbefüllbar ausgebildet ist.

15. Inhalationsvorrichtung nach einem der vorstehenden Ansprüche, **dadurch gekennzeichnet, dass**
die Inhalationsvorrichtung (10; 110; 210; 310) eine im Wesentlichen längliche Struktur aufweist, wobei das Mundstück (82; 128; 282) an einem proximalen Ende der Inhalationsvorrichtung und die Heizeinrichtung (50; 350) im Bereich des distalen Endes der Inhalationsvorrichtung angeordnet ist.

## Claims

1. An inhalation device (10; 110; 210; 310) for the inhalation administration of an inhalation mixture of air and at least one additive material, comprising
- a mouthpiece (82; 182; 282),
- an inhalation mixture generator (70; 170; 270; 370) with an air inlet opening (72; 272),
- a heating unit (50; 350) for combusting a fuel (258) and for heating the air and/or the additive material and/or the inhalation mixture,
- the inhalation mixture generator (70; 170; 270; 370) being connected to the mouthpiece (82; 182; 282) directly or via an inhalation duct,
**characterized in that**
the heating unit (50; 350) comprises
- a fuel storage (52; 152; 252) that is spatially separate from the inhalation mixture generator and can be filled or is filled with a thermally combustible solid or liquid fuel (258), and
- a combustion chamber (56; 156; 256) that is designed to form a flame to combust the fuel (258) and is largely closed off from the surroundings by a combustion chamber wall (22; 122; 222) that forms an outside wall of the inhalation device,
- at least part of the combustion chamber wall (22; 122; 222) having micro openings (24; 124; 224) that are designed such that
- the sum of the outer side lengths of all micro openings (24; 124; 224) is at least 140 mm and
- the sum of the outer side lengths of the micro openings (24; 124; 224) per surface in the area of the combustion chamber wall (22; 122; 222) averages at least 80 mm/cm².

2. The inhalation device according to claim 1, **characterized in that**
a majority of the micro openings (24; 124; 224) is provided on a wall (20; 120; 220; 320) that is lateral relative to a main direction of extent of the inhalation device.

3. The inhalation device according to one of claims 1 or 2, **characterized in that**
- the sum of the outer side lengths of all micro openings (24; 124; 224) is at least 700 mm and
- the sum of the outer side lengths of the micro openings (24; 124; 224) per surface in the area of the combustion chamber wall (22; 122; 222) averages at least 400 mm/cm².

4. The inhalation device according to one of the preceding claims, **characterized in that**
the micro openings (24; 124; 224) have an average aperture surface of less than 1.5 mm².

5. The inhalation device according to one of the preceding claims, **characterized in that**
an area of the combustion chamber wall (22; 122; 222) that is open due to the micro openings (24; 124; 224) occupies between 10% and 50% of a section of the combustion chamber wall (22; 122; 222) that is provided with the micro openings (24; 124; 224).

6. The inhalation device according to one of the preceding claims, **characterized in that**
the combustion chamber wall is formed at least partly by a metal fabric or metal braid of metal wires.

7. The inhalation device according to one of the preceding claims, **characterized in that**
the combustion chamber wall (22; 122; 222) is formed at least partly by a metal foil with incorporated micro openings (24; 124; 224).

8. The inhalation device according to one of the preceding claims, **characterized in that**
the combustion chamber (56; 156; 256) is accessible from outside only in the area of an ignition access (26; 126; 236).

9. The inhalation device according to one of the preceding claims, **characterized in that**
the fuel (258) consists at least partly of one or more of the substances selected from the following group: alcohols, aldehydes, ketones, esters, n-alkanes with one to four carbon atoms, n-alkanes with five to twenty carbon atoms, branched or cyclic alkanes with four to twenty carbon atoms.

10. The inhalation device according to one of the preceding claims, **characterized in that**
the fuel storage (152) is closed by means of a gastight closure that can be removed by heating.

11. The inhalation device according to one of the preceding claims, **characterized in that**
the fuel storage (252) is closed by means of a gastight closure (260) that can be removed or torn open by a pressure that can be applied to the fuel storage manually, preferably by pressure on the inhalation device from both sides in the area of the fuel storage.

12. The inhalation device according to one of the preceding claims, **characterized in that**
the fuel storage (252) can be connected releasably to the heating unit.

13. The inhalation device according to one of the preceding claims, **characterized in that**
the heating unit (350) is designed as a device that can be handled separately and that can be connected releasably to the inhalation mixture generator (370).

14. The inhalation device according to one of the preceding claims, **characterized in that**
an additive material storage that is designed separate from the inhalation mixture generator is provided, the additive material storage being designed so as to be replaceable or refillable.

15. The inhalation device according to one of the preceding claims, **characterized in that**
the inhalation device (10; 110; 210; 310) has an essentially elongate structure, the mouthpiece (82; 128; 282) being arranged at a proximal end of the inhalation device and the heating unit (50; 350) being arranged in the area of the distal end of the inhalation device.

## Revendications

1. Dispositif d'inhalation (10; 110; 210; 310) pour la prise par inhalation d'un mélange à inhaler constitué d'air et d'au moins un additif avec
- une embouchure (82; 182; 282),
- un producteur de mélange à inhaler (70; 170; 270; 370) avec une ouverture d'admission d'air (72; 272),
- une unité de chauffage (50; 350) pour brûler un combustible (258) et pour chauffer l'air et/ou l'additif et/ou le mélange à inhaler,
auquel cas
- le producteur de mélange à inhaler (70; 170; 270; 370) est directement relié à l'embouchure (82; 182; 282) ou par l'intermédiaire d'un canal d'inhalation,
**caractérisé par le fait que**
l'unité de chauffage (50; 350)
- présente un réservoir de combustible (52; 152; 252) séparé du producteur de mélange à inhaler qui est ou peut être rempli d'un combustible (258) solide ou liquide, et
- une chambre de combustion (56; 156; 256) destinée à la formation d'une flamme pour brûler le combustible (258) qui est essentiellement isolée de l'environnement par une paroi (22; 122; 222) de chambre de combustion formant une paroi extérieure du dispositif d'inhalation,
auquel cas
- la paroi (22; 122; 222) de chambre de combustion présente au moins pour partie des micro-ouvertures (24; 124; 224) qui sont formées de sorte que
- la somme des longueurs de bords de toutes les micro-ouvertures (24; 124; 224) soit d'au moins 140 mm et que
- la somme des longueurs de bords des micro-ouvertures (24; 124; 224) par unité de surface dans le secteur de la paroi (22; 122; 222) de chambre de combustion soit d'au moins 80 mm / cm² en moyenne.

2. Dispositif d'inhalation suivant la revendication 1,
**caractérisé par le fait que**
les micro-ouvertures (24; 124; 224) sont en majeure partie prévues sur une paroi (20; 120; 220; 320) latérale par rapport à une orientation principale du dispositif d'inhalation.

3. Dispositif d'inhalation suivant l'une des revendications 1 ou 2, **caractérisé par le fait que**
- la somme des longueurs de bords de toutes les micro-ouvertures (24; 124; 224) soit d'au moins 700 mm et que
- la somme des longueurs de bords des micro-ouvertures (24; 124; 224) par unité de surface dans le secteur de la paroi (22; 122; 222) de chambre de combustion soit d'au moins 400 mm / cm² en moyenne

4. Dispositif d'inhalation suivant l'une des revendications ci-dessus, **caractérisé par le fait que**
les micro-ouvertures (24; 124; 224) présentent une surface d'ouverture moyenne inférieure à 1,5 mm².

5. Dispositif d'inhalation suivant l'une des revendications ci-dessus, **caractérisé par le fait que**
un secteur de la paroi de la chambre de combustion (22; 122; 222) ouvert par des micro-ouvertures (24; 124; 224) occupe de 10% et 50% d'une section de la paroi de la chambre de combustion (22; 122; 222) pourvue de micro-ouvertures (24; 124; 224).

6. Dispositif d'inhalation suivant l'une des revendications ci-dessus, **caractérisé par le fait que**
la paroi de la chambre de combustion est au moins en partie formée d'un tissu métallique ou d'un treillis de fils métalliques.

7. Dispositif d'inhalation suivant l'une des revendications ci-dessus, **caractérisé par le fait que**
la paroi de la chambre de combustion (22; 122; 222) est au moins en partie formée d'une feuille métallique dans laquelle sont pratiquées des micro-ouvertures (24; 124; 224).

8. Dispositif d'inhalation suivant l'une des revendications ci-dessus, **caractérisé par le fait que**
la chambre de combustion (56; 156; 256) n'est accessible de l'extérieur qu'au niveau d'un accès d'allumage (26; 126; 236).

9. Dispositif d'inhalation suivant l'une des revendications ci-dessus, **caractérisé par le fait que**
le combustible (258) se compose, du moins en partie, d'une ou plusieurs substances choisies dans les groupes suivants : alcools, aldéhydes, cétones, esters, alcanes linéaires avec de un à quatre atomes de carbone, alcanes linéaires avec de cinq à vingt atomes de carbone, alcanes ramifiés ou annulaires avec de quatre à vingt atomes de carbone.

10. Dispositif d'inhalation suivant l'une des revendications ci-dessus, **caractérisé par le fait que**
le réservoir de combustible (152) est fermé à l'aide d'un bouchon étanche au gaz qui se laisse retirer par chauffage.

11. Dispositif d'inhalation suivant l'une des revendications ci-dessus, **caractérisé par le fait que**
le réservoir de combustible (252) est fermé à l'aide d'un bouchon (260) étanche au gaz qui se laisse retirer ou arracher par une pression applicable manuellement sur le réservoir de combustible, de préférence en exerçant une pression des deux côtés sur le dispositif d'inhalation au niveau du réservoir de combustible.

12. Dispositif d'inhalation suivant l'une des revendications ci-dessus, **caractérisé par le fait que**
le réservoir de combustible (252) peut être relié de façon amovible avec l'unité de chauffage.

13. Dispositif d'inhalation suivant l'une des revendications ci-dessus, **caractérisé par le fait que**
l'unité de chauffage (350) est formée comme dispositif manipulable séparément qui peut être relié de façon amovible avec le producteur de mélange à inhaler (370).

14. Dispositif d'inhalation suivant l'une des revendications ci-dessus, **caractérisé par le fait que**
un réservoir d'additif formé séparément du producteur de mélange à inhaler est prévu, auquel cas le réservoir d'additif peut être échangé ou de nouveau rempli.

15. Dispositif d'inhalation suivant l'une des revendications ci-dessus, **caractérisé par le fait que**
le dispositif d'inhalation (10; 110; 210; 310) présente une structure globalement oblongue, auquel cas l'embouchure (82; 128; 282) est disposée à une extrémité proche du dispositif d'inhalation, et l'unité de chauffage (50; 350) au niveau de l'extrémité distale du dispositif d'inhalation.
